# EUROPEAN PATENT SPECIFICATION

(11) **EP 4 637 504 B1**
(45) Date of publication and mention of the grant of the patent: **09.12.2026**
(21) Application number: 23834045.9
(22) Date of filing: 18.12.2023
(51) Int. Cl.: A61B 1/005, A61M 25/01

(54) **DEFLECTION CONTROL MECHANISM FOR A MEDICAL DEVICE, MEDICAL DEVICE, METHOD FOR SERVICING AND METHOD FOR ASSEMBLING OF A DEFLECTION CONTROL MECHANISM OR A MEDICAL DEVICE**
ABLENKSTEUERUNGSMECHANISMUS FÜR EIN MEDIZINISCHES GERÄT, MEDIZINISCHES GERÄT, WARTUNGSVERFAHREN UND VERFAHREN ZUR MONTAGE EINES ABLENKSTEUERUNGSMECHANISMUS ODER EINES MEDIZINISCHEN GERÄTS
MÉCANISME DE COMMANDE DE DÉVIATION POUR DISPOSITIF MÉDICAL, DISPOSITIF MÉDICAL, PROCÉDÉ D'ENTRETIEN ET PROCÉDÉ D'ASSEMBLAGE DE MÉCANISME DE COMMANDE DE DÉVIATION OU DE DISPOSITIF MÉDICAL

(30) Priority: 20.12.2022 DE 102022134198
(43) Date of publication of application: 29.10.2025
(73) Proprietor: KARL STORZ SE & Co. KG, 78532 Tuttlingen (DE)
(72) Inventor: MONAKOV, Igor, 78532 Tuttlingen (DE); TOMBERG, Johan, 78532 Tuttlingen (DE)
(86) International application number: PCT/EP2023/086304
(87) International publication number: WO 2024/133047

(56) References cited:
- WO-A1-2022/008442
- WO-A1-2022/008445
- JP-A- H0 265 831
- US-A- 4 905 666
- US-A1- 2013 038 930

## Description

The present invention relates to a deflection control mechanism for a medical device, particularly for a steerable flexible endoscope, the endoscope having an elongate flexible shaft comprising a steerable section that is deflectable by movement of at least one pair of counteracting control wires, the deflection control mechanism comprising a support structure, a drive wheel rotatably supported in or on the support structure, and an elongate traction element having a flexible section, a first coupling section connected to a first end of the flexible section and a second coupling section connected to a second end of the flexible section, wherein the flexible section of the traction element mechanically engages with the drive wheel, wherein the first and second coupling sections each comprise a fixation element for fixing a respective proximal end of each control wire of the at least one pair of control wires, whereas each control wire features a respective guide tube to guide each respective wire along a longitudinal axis, the respective guide tube being held in a respective guide tube mount opening of a guide tube mount of the support structure, the guide tube mount opening having an entry opening to accept the respective guide tube. Furthermore, the invention relates to a medical device, particularly a steerable flexible endoscope with such a deflection control mechanism, a method for servicing a deflection mechanism or a medical device and a method for assembling of a deflection control mechanism or a medical device.

Flexible endoscopes comprise a flexible elongate shaft that is configured for being inserted into an internal cavity of a human or animal body or any other object. In a distal (for example distant from a user) end section of the shaft, an imaging optics is arranged for generating an image of a scene in the cavity being observed. The endoscopic image generated may be transmitted to a proximal (for example close to the user) end of the shaft by a bundle of optical fibers to be picked up by an electronic image sensor positioned in a handpiece arranged at the proximal end of the shaft, or an electronic image sensor may be arranged in the distal end section of the shaft, the image signal being transmitted by electric lines arranged inside the shaft. A flexible endoscope usually comprises an illumination system for illuminating the cavity to be observed, and one or more instrument and/or fluid channels extending from a handpiece through the shaft to its distal end.

Flexible endoscopes are typically employed for observing a cavity being accessed through a curved path, or to observe the interior of an organ that itself has a curved shape, such as the intestine, for example. Due to its flexibility, the flexible shaft is able to adapt during insertion to the curvature of the access path or the organ. Frequently, however, it is desirable to be able to actively deflect or bend the distal end section of the flexible shaft out of a straight alignment into a bent or articulated shape, in order to facilitate insertion through a curved access path or organ. Moreover, as a viewing direction of the imaging optics generally is determined by a direction of the distal end section of the endoscope shaft, active deflection of the distal end section permits observing a desired partial area of the cavity, and may permit substantially complete observation of a surface of the cavity by varying a deflection angle of the distal end section. Endoscopes permitting active deflection or bending of a section of the flexible shaft are usually denoted "steerable endoscopes".

A steerable or deflectable section of the shaft of a steerable endoscope typically has a supporting structure of consecutively jointed pivoting elements, for example, wherein the pivoting elements can be tipped toward one another by axially moveable control wires. Alternatively, the supporting structure may be formed by one or more elastic bending elements, for example, which can be bent by the action of control wires. The supporting structure is enclosed in a flexible sheath made of a synthetic material, for example. Deflection of the steerable section from an aligned position into a bent or articulated shape in one or an opposite direction can be controlled by a reciprocating movement of one pair of counteracting control wires. Frequently, steerable endoscopes permit bending the steerable section into perpendicular planes by operation of two pairs of control wires. The control wires may be fed along the outside or inside of the pivoting elements and extend through the shaft in a proximal direction into the handpiece where a deflection control mechanism is provided. The deflection control mechanism effects movement of the control wires under user control, for example by turning one or more handwheels.

Due to the design of the pivoting elements or the elastic bending elements and/or due to requirements of the flexible sheath or other elements of the shaft having a minimal bending radius, articulation of the steerable section is limited to a maximal permissible deflection angle. During operation of the steerable endoscope, however, a user normally has no direct visual control of the articulation of the steerable section. Thus, steerable endoscopes have been provided having a deflection control mechanism that comprises a mechanical stop, in order to avoid exceeding the maximal permissible deflection angle and thus to avoid breakage or inadmissible deformation of components.

Especially for servicing a deflection control mechanism or a respective medical device or for assembling such a mechanism or device, usually the sheaths and/or the control wires have to be mounted at or connected to the support structure, which is usually done by screwing those sheaths down into a holding position and/or gluing or welding those sheaths to the support structure, which is inconveniently inflexible for both assembling and later service operations. In either case, an extensive amount of work is needed to disassemble and service such a deflection control mechanism and thus such a medical device.

WO 2022/008445 A1 discloses a deflection control mechanism for a steerable flexible endoscope whose support structure comprises a frame element with lateral cutouts that each hold permanently a wire coil mounting, the wire coil mountings retaining wire coils that guide the control wires whose proximal ends are fixed to coupling sections engaging a drive wheel via a traction element.

It is therefore an objective of the present invention to improve this state of the art.

This objective is met by a deflection control mechanism for a medical device, particularly for a steerable flexible endoscope, the endoscope having an elongate flexible shaft comprising a steerable section that is deflectable by movement of at least one pair of counteracting control wires, the deflection control mechanism comprising a support structure, a drive wheel rotatably supported in or on the support structure and an elongate traction element having a flexible section, a first coupling section connected to a first end of the flexible section and a second coupling section connected to a second end of the flexible section, wherein the flexible section of the traction element mechanically engages with the drive wheel, wherein the first and second coupling section each comprise a fixation element for fixing a respective proximal end of each control wire of the at least one pair of control wires, whereas each control wire features a respective guide tube to guide each respective control wire along a longitudinal axis, the respective guide tube being held in a respective guide tube mount opening of a guide tube mount of the support structure, the guide tube mount opening having an entry opening to accept the respective guide tube, wherein the respective guide tube mount opening features a clamping device with a respective clamping lever, the clamping lever being linked to the guide tube mount with a spring mount and thus being elastically moveable against a restoring force of the spring mount in relation to the guide tube mount opening between a clamping position to hold the respective guide tube and the respective guide tube mount opening by reducing a size of guide tube mount opening and/or applying the restoring force to the respective guide tube and a mounting position to release the respective guide tube from the guide tube mount opening by being elastically expanded against the restoring force to expand the size of the guide tube mount opening.

This clamping device with a respective clamping lever enables for an easy attachment and detachment of each guide tube and thus for easy service as well as easy assembly of a respective deflection control mechanism or a respective medical device. Instead of having a complicated attachment procedure to attach each guide tube to the respective guide tube mount opening, each guide tube can easily be pushed through the respective entry opening to arrive in the guide tube mount opening and be held by the respective clamping lever and its restoring force.

As utilized in accordance with the present disclosure, the following terms, unless otherwise indicated, shall be understood to have the following meanings:

A "deflection control mechanism" especially serves as a mechanical arrangement for controlling the respective deflection of, for example, a flexible endoscope, whereas the deflection control mechanism as such features all the necessary mechanical means to accomplish its task. In this respect, a "medical device" is a device which is suitable for medical purposes, particularly such a medical device is a so-called "steerable flexible endoscope", which serves as an optical instrument to be fed into a cavity and as such being able to give a user a detailed view of such cavity.

An endoscope features an "elongate flexible shaft" which is a slim and relatively long part of the endoscope, being fed through an entry of the above-mentioned cavity and as such being flexible for being steered into a suitable direction, for example to align said direction with a feasible direction of view. This elongate flexible shaft features a "steerable section", which is deflectable by the deflection control mechanism and as such can be steered into a respective direction. For this deflection, at lest one pair of counteracting "control wires", which can, for example, be steel or stainless-steel wires being counteractingly attached to the steerable section, is used to mechanically steer the steerable section.

A "support structure" which serves as a main structure or mechanical attachment structure of the deflection control mechanism holds a "drive wheel" which serves as a manual interface and is rotatably supported in or on the support structure. Along with the drive wheel, an "elongate traction element", for example a chain or a rope or a similar means, is engaged with the drive wheel with a flexible section and features a respective coupling section to be attached to a respective proximal end of each control wire to manipulate said control wires with the drive wheel respectively. "Proximal" in this respect describes an orientation towards an operator, whereas "distal" refers to a respective opposite direction which is usually located away from an operator, both in view of a usual operating position of the medical device.

Each control wire features a respective "guide tube", whereas each guide tube, for example a plastic tube or plastic hose of certain stability, serves as a guide for each respective control wire and as such ensures for a longitudinal movement of each control wire along a longitudinal axis, whereas any other movement shall be restricted by the shape and diameter of each guide tube. Such a diameter, for example, can be chosen just slightly above an outer diameter of each respective control wire for enabling for longitudinal movement and reducing possible slack within usual technical tolerances.

Each guide tube is held in a respective "guide tube mount opening", whereas the latter is a physical opening in the support structure and/or a "guide tube mount" of the support structure to accommodate the respective guide tube in one, two or three axes. An "entry opening" of the guide tube mount opening is especially orientated orthogonally to the longitudinal axis of each control wire and respectively of each guide tube such that the respective guide tube can be entered into the guide tube mount opening sideways in relation to the longitudinal axis through said entry opening.

A "clamping device", being designed to hold each guide tube by elastic deformation and forces stored in that elastic deformation of, for example, a part of the support structure or a part of the guide tube mount, features a "clamping lever" which is a slim and relatively long element in relation to its width and as such being linked to the guide tube mount with a so-called "spring mount", which can either be designed as a material spring using elastic energy stored in deformed material or a separate spring system supporting the clamping lever elastically. As such, the clamping lever incorporates a "restoring force" when deformed from its ideal position in relation to the guide tube mount opening, which can also incorporate a remaining restoring force while holding the respective guide tube. A "clamping position" is a position that holds the respective guide tube in the respective guide tube mount opening by reducing a size of the guide tube mount opening, for example by being positioned against the respective guide tube by part of the restoring force and as such holding the guide tube. When deformed, the clamping lever transfers into a "mounting position", which geometrically enables for releasing the respective guide tube from the guide tube mount and, in parallel, enlarging the restoring force of the respective clamping lever.

As such, the "clamping lever" respectively serves as a spring-loaded clamping means to hold each guide tube with resulting restoring force of the spring system.

For enabling for easy manufacturing, the clamping lever is integrally formed into the guide tube mount by a gap, the gap separating a main body of the guide tube mount from the clamping lever. Thus, the clamping lever can, for example, be manufactured within the main body of the guide tube mount, reducing the necessary amou8nt of parts.

"Integrally formed" in this respect refers to the clamping lever being part of the same material and the same workpiece to, in the next manufacturing step, forming the guide tube mount. Such workpiece features a so-called "main body" which is separated from the clamping lever by a "gap", respectively a lack of material located in between the main body and the clamping lever.

According to a further embodiment of the invention, a spring section of the clamping lever forms the spring mount by using elastic material properties of a material of the clamping lever and/or of the guide tube mount.

As already mentioned above, these elastic material properties of a material forming the guide tube mount and/or the clamping lever can be used for forming or presenting a so-called "spring section", as such being a section of the clamping lever acting as a spring. As such, the clamping lever can feature a comparably slim part forming the spring section in contrast to a, for example, comparably thicker part of the clamping lever forming the clamping lever itself.

For ensuring a tight and safe clamping operation towards the respective guide tube, the respective clamping lever features a locking section, the locking section protruding from the clamping lever into the guide tube mount opening to locally reduce the size of the guide tube mount opening for locking the respective guide tube in the clamping position.

Such a "locking section" is, e.g. assuming a round or elliptic guide tube, located out of axis in relation to the longitudinal axis and as such serves as a mechanical locking by protruding from the clamping lever and thus locally reducing the size of the guide tube mount opening.

According to a further embodiment of the invention, the respective clamping lever and/o the respective guide tube mount features a chamfer positioned at the entry opening to facilitate accepting the respective guide tube by reducing the necessary force for entering the respective guide tube into the guide tube mount opening against the restoring force.

Such a "chamfer" opens a diameter of the respective guide tube mount opening towards a direction out of which the guide tube is pushed into the guide tube mount opening through the entry opening and as such assists in widening such guide tube mount opening locally by, for example, moving the clamping lever out of the way for accepting the guide tube.

For restricting a rotary motion of each guide tube inherently, the guide tube mount opening features an uneven geometry with varying radii around its circumference such that an uneven geometry of the respective guide tube matches the uneven geometry of the guide tube mount opening in the clamping position to prevent the guide tube from rotating around the longitudinal axis.

Such an "uneven geometry", which is, for example, a geometry deviating from a round shape and as such can be oval, partly rectangular or in another way differently shaped, geometrically prevents each guide tube from rotating around the longitudinal axis. In this respect, the respective uneven geometry of the respective guide tube can either be inherently built into or onto the guide tube or can also be achieved by deforming the guide locally to match the uneven geometry of the guide tube mount opening.

In yet another embodiment of the invention, the respective guide tube features a longitudinal stop engaging with the guide tube mount in the clamping position to prevent the guide tube from moving in relation to the guide tube mount along the longitudinal axis.

Such a "longitudinal stop" can, for example, be a mechanical bushing or a mechanical extension of the guide tube or a part attached to the guide tube as well as an insertion of the respective guide tube being positioned against the guide tube mount mechanically and as such at least preventing the guide tube from moving in relation to the guide tube mount along the longitudinal axis in at least one direction.

For enabling for an easy manufacturing, the guide tube features a locking piece being mechanically attached to the guide tube, especially presenting the uneven geometry of the guide tube and/or the longitudinal stop.

As such, a "locking piece", which is, for example, a separate piece being attached to the guide tube, can be glued, welded or pressed on the respective guide tube, e.g. prior to the assembly process.

According to a further embodiment of the invention, the guide tube mount is formed into a first mount plate, which is especially positioned essentially orthogonally in relation to the longitudinal axis, whereas the guide tube mount especially features a second mount plate, a third mount plate and/or another mount plate, each positioned essentially orthogonally in relation to the longitudinal axis and forming the guide tube mount, the uneven geometry and/or the longitudinal stop.

Such a "mount plate", for example a flat mount plate, can be easily manufactured and positioned in relation to the longitudinal axis, especially on the support structure. In case of the use of multiple mount plates, for example a second mount plate, a third mount plate and/or further mount plates, each mount plate can be similar or equal to the respective other mount plate or feature respective capabilities. As an example, the first mount plate can serve as the clamping device, featuring the respective clamping lever or clamping levers, while a second mount plate or a third mount plate serve as the longitudinal stop. Other embodiments are possible, of course.

For an easy attachment and assembly, the support structure features support structure elements with hooking means to accept the guide tube mount, especially the first mount plate, the second mount plate, the third mount plate and/or the other mount plate, whereas the hooking means especially accept the guide tube mount, especially the first mount plate, the second mount plate, the third mount plate and/or the other mount plate in a hooking axis being essentially positioned in parallel to a mount plate of the guide tube mount, the first mount plate, the second mount plate, the third mount plate and/or the other mount plate.

In yet another embodiment of the invention, the guide tube mount, the first mount plate, the second mount plate, the third plate and/or the other mount plate and/or the support structure element is and/or are manufactured from steel, especially from stainless-steel, and/or from sheet material, especially cut via laser cutting and/or waterjet cutting.

For easy assembly as well as protection of parts of the deflection control mechanism, the support structure features a cover. The cover is engaging with the support structure in a covering position to protect the support structure, the elongate traction element, the first coupling section, the second coupling section, the respective fixation element and/or a respective control wire and/or fixing the support elements and/or the guide tube mount, the first mount plate, the second mount plate, the third mount plate and/or the other mount place in place, whereas especially the respective guide tube or the respective guide tubes and/or the locking piece is held in its position in the clamping position by the cover in the covering position.

Especially when the cover serves as a positioning device in this respect, the parts within the cover, for example each guide tube and/or each locking piece, can be held in position both along transfer axes as well as rotary positions. As such, the cover can also serve as geometrical means for avoiding or preventing a rotary motion of each guide tube around the longitudinal axis.

According to another aspect, the problem is solved by a medical device, particularly a steerable flexible endoscope, with a deflection control mechanism according to one or more of the above-mentioned embodiments. This medical device can be easily serviced and/or assembled.

According to a further aspect, the problem is solved by a method for servicing a deflection control mechanism or a medical device according to one of the above-mentioned embodiments, comprising the following steps:
- Disengaging the respective guide tube from the respective guide tube mount opening from the clamping position by retracting the respective guide tube against the restoring force such that the respective guide tube is free from the respective guide tube mount,
- Manipulating the respective fixation element and/or the respective control wire according to a service operation, such that the service operation has been performed,
- Reengaging the respective guide tube with the respective guide tube mount opening into the clamping position by pushing the respective guide tube into the respective guide tube mount opening against the restoring force such that the respective guide tube is brought into the clamping position and thus being fixed in the respective guide tube mount opening, such that the deflection control mechanism or the medical device is being serviced.

In this respect, it has to be mentioned that a service operation for a deflection control mechanism or a medical device, especially a flexible endoscope, often comprises the readjustment of a tension of each control wire and as such needs as partial disassembling of the deflection mechanism itself. According to the method for servicing a deflection control mechanism or a medical device as described above, the guide tube can easily be disengaged from the clamping position by pulling the guide tube out of the guide tube mount opening by working against the restoring force and as such taking advantage from the flexibility of each clamping lever. After the manipulation of the respective part of the mechanism, each guide tube can easily be pushed into the guide tube mount opening again by taking advantage of the elastic feature of each clamping lever.

"Manipulating" the respective fixation element and/or the respective control wire in this respect refers to, for example, readjusting a threaded portion of the control wire or the fixation element for readjusting a specific tension of each control wire. Also, manipulating can refer to any other servicing operation, such as oiling a respective control wire and/or guide tube, for example.

In yet another aspect, the problem is solved by a method for assembling a deflection control mechanism according to one of the above-mentioned embodiments or a medical device according to the above-mentioned embodiment, comprising the following steps:
- Presenting the support structure, at least one guide tube and the guide tube mount, such that the support structure, at least one guide tube and the guide tube mount are present,
- Engaging the respective guide tube with the respective guide tube mount opening into the clamping position by pushing the respective guide tube into the respective guide tube mount opening against the restoring force such that the respective guide tube is brought into the clamping position and thus being fixed in the respective guide tube mount opening,
such that the deflection control mechanism or the medical device is assembled.

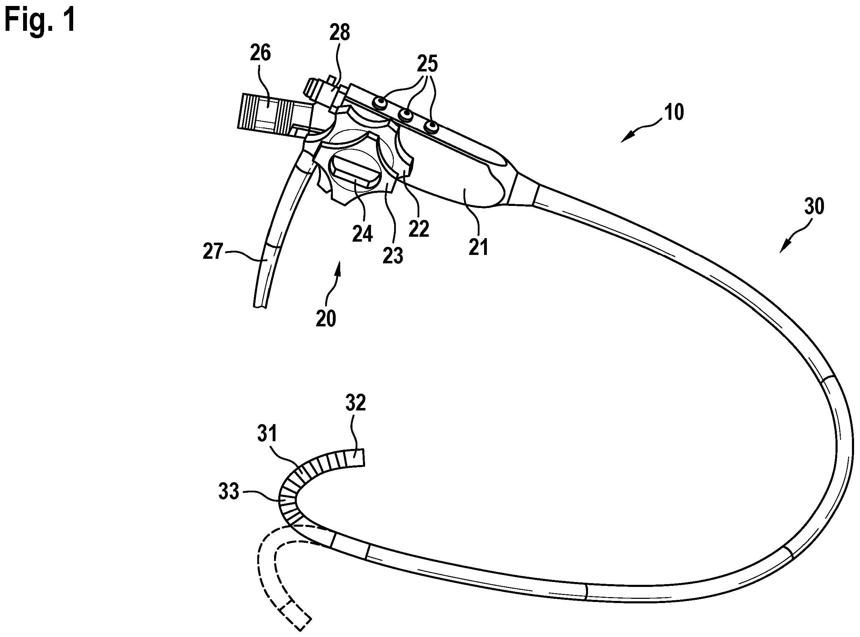

Further aspects of the present invention will be apparent from the figures and from the description of particular embodiments as
- Fig. 1: shows a steerable flexible endoscope in an overall view,

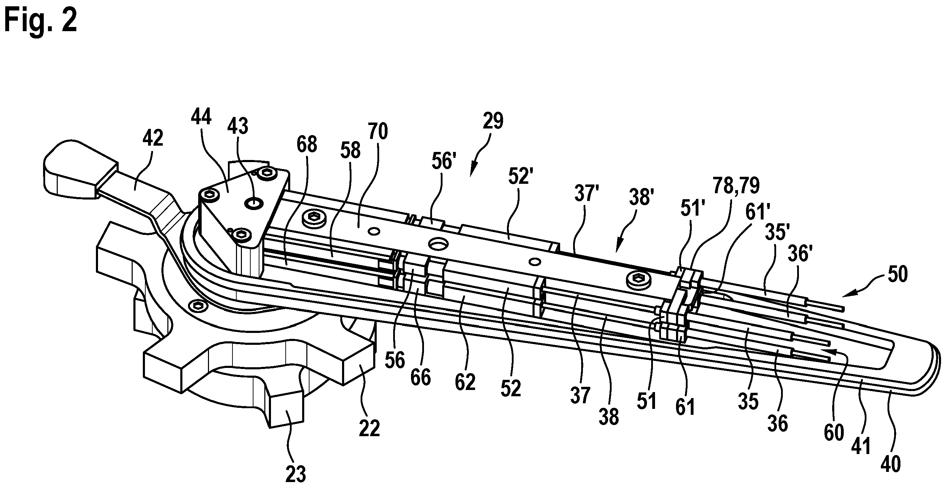

- Fig. 2: shows a deflection control mechanism in accordance with an exemplary embodiment of the present invention,

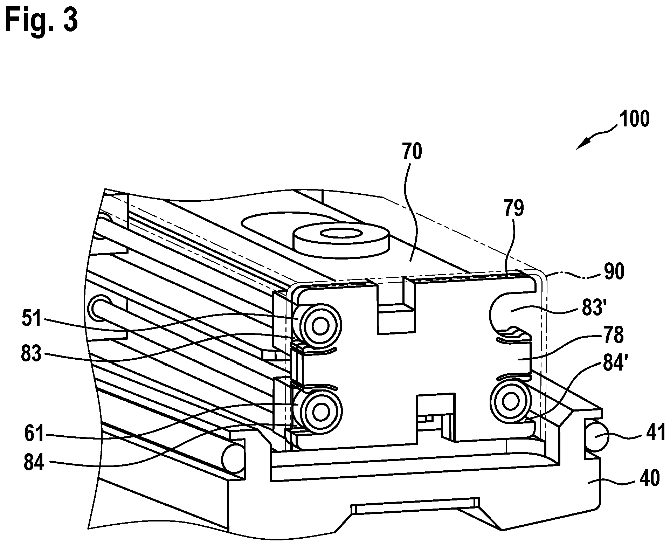

- Fig. 3: shows a front section of the deflection control mechanism, specifically a holder in an isometric view,
- Fig. 4a: shows a further detailed view of the detail of Fig. 3,
- Fig. 4b: shows the detailed view of Fig. 4a in a cross-sectional front view,
- Fig. 5: shows an isometric view of an optional holder at a front section of the deflection control mechanism,
- Fig. 6: shows a further option for a front section with an optional holder,

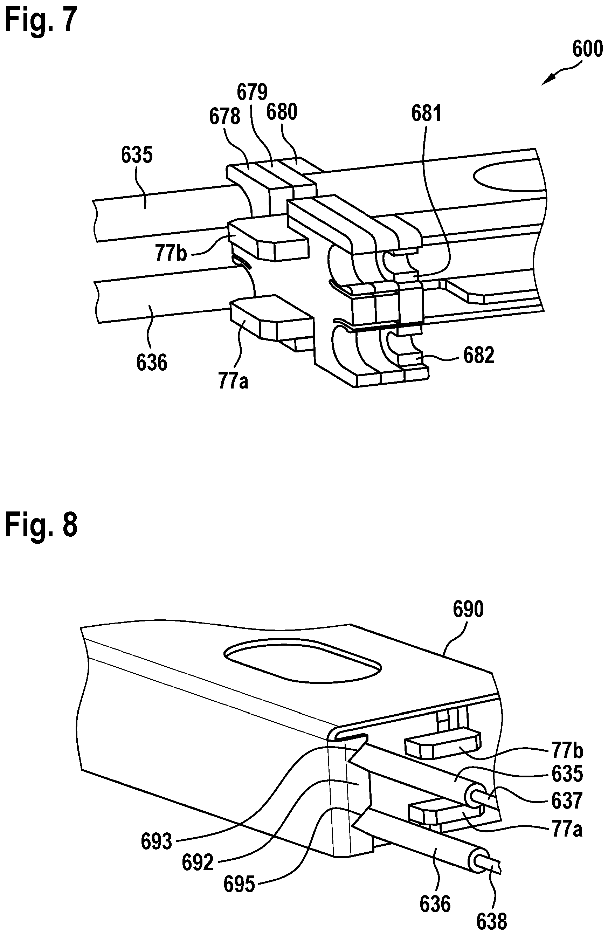

- Fig. 7: shows the optional holder of Fig. 6 in a different, isometric view,
- Fig. 8: shows the holder and front section of the deflection control mechanism of Figs. 6 and 7 with a cover, and
- Fig. 9: shows a further optional holder for the front section of the deflection control mechanism.

A flexible endoscope 10 typically comprises a handpiece 20 and an elongate flexible shaft 30, the handpiece 20 being attached to a proximal end of the shaft 30. The handpiece 20 has an outer housing 21 made of plastic and/or metallic material. On a lower side of the housing 21, a first handwheel 22 and a second handwheel 23 are arranged for controlling a deflection of a steerable section 31 of the shaft 30, as is described below. Typically, the first and second handwheel 22, 23 are arranged coaxially, and, at an exterior side of the second handwheel 23, a knob 24 for controlling a deflection break relating to the second handwheel 23 is provided. Further, the handpiece 20 may exhibit a multiplicity of control buttons 25 for controlling various functions of the flexible endoscope 10, such as for controlling the imaging and/or illumination system and/or irrigation and suction pumps, for example. The handpiece 20 may be connectable to an external video unit or a video monitor via a connector 26 and to an external light source via light cables 27. Moreover, an instrument port 28 may be provided for inserting endoscopic instruments to be advanced through one or more respective channels to a distal end of the shaft 30 for manipulating tissue or other optics within a cavity into which the shaft 30 can be inserted.

At its distal end, the flexible shaft 30 comprises a steerable section 31. The steerable section 31 may form a distal end section of the shaft 30 or a carrier distal end cap 32 which may accommodate an imaging optics and an electronic image sensor for providing an endoscopic image of a cavity into which the shaft 30 is inserted. The image signal generated by the image sensor may be transmitted via electric cables extending through the shaft 30 and the handpiece 20 to the connector 26 for being processed and displayed by an external video unit. The shaft 30 in total is flexible to an extend to be advanced through an endoscopic access or a hollow organ towards a cavity to be observed, being capable of adapting to a curved shaped of the access or the organ. The steerable section 31, on the other hand, is capable of being flexed actively by turning the handwheels 22, 23. To this end, the steerable section 31 comprises an inner structure of a multiplicity of consecutive pivoting elements 33, thus being deflectable in one or more planes. The pivoting elements 33 are covered by a flexible tube to form a smooth outer surface, the shaft 30 in total having a uniform cross-sectional shape and diameter.

For controlling the deflection of the steerable section 31, a set of two counteracting control wires are provided extending on opposites sides within the steerable section 31, by a longitudinal movement of which the steerable section 31 can be bent to one or the other side. The control wires, namely the control wires 37, 37', 38 and 38', extend along the shaft 30 and are connected at their proximal ends to a deflection control mechanism arranged within the handpiece 20 which can be operated by a user by turning the handwheels 22, 23 for deflecting the steerable section 31. In this respect, the respective numerals, for example 37 and 37', 38 and 38' and so on for further elements, refer to respective pairs of elements, for example a pair of control wires 37 and 37' both responsible for deflecting the steerable section 31 in a respective plane.

An internal portion of the handpiece 20 of the flexible endoscope 10 is built on a body 40 which accommodates the respective parts and features a seal ring 41 to sealingly engage with the housing 21.

The body 40 is preferably formed of a metal, such as stainless-steel, for example. A deflection control mechanism 29 is held by the body 40, wherein, at a lower side of the body 40, the first handwheel 22 and the second handwheel 23 are rotatably mounted, having a common axis of rotation. The common axis is defined by an axle 43 that is non-rotating and fixedly held in a rigid metal cage 44. The axle defines a rotation axis of each drive wheel that is rotationally coupled with the first handwheel and the second handwheel 22, 23. A lever 42 is provided for controlling a deflection break relating to the first handwheel 22.

Each sprocket (not shown) engages with a respective chain 58 and 68, respectively connected to the control wires via blocks 56 and 56', respectively 66 and 66', each threaded into a respective block 52 and 52' and 62 and 62' being attached to a respective end of each control wire. Thus, each handwheel 22, 23 mechanically engages with the respective control wires. Each control wire 37, 37', 38, 38' is guided in a respective sheath 35, 35', 36, 36' to be led towards the steerable section 31 of the shaft 30.

To accommodate the respective sheaths 35, 35', 36, 36' against the main structure of the deflection control mechanism 29, covered by a plastic plate 70, the respective sheaths 35, 35', 36, 36' are held in frame plates 78 and 79 which are mechanically engaged with the structural elements of the deflection control mechanism 29. Therefore, each sheath 35, 35', 36, 36' features a respective bushing 51, 51', 61, 61' to be mechanically linked in the respective frame plates 78 and 79.

The frame plates 78 and 79, forming a holder 100, feature cut-outs 83, 83', 84' and 84' symmetrically organized in the cross-section of the frame plates 78 and 79. Each cut-out is, in general, U-shaped and as such open to the outside of the assembly for accepting the respective bushing 51, 51', 61, 61'. While completely assembled, a cover 90 covers the deflection control mechanism 29 and holds each bushing 51, 51', 61, 61' in position.

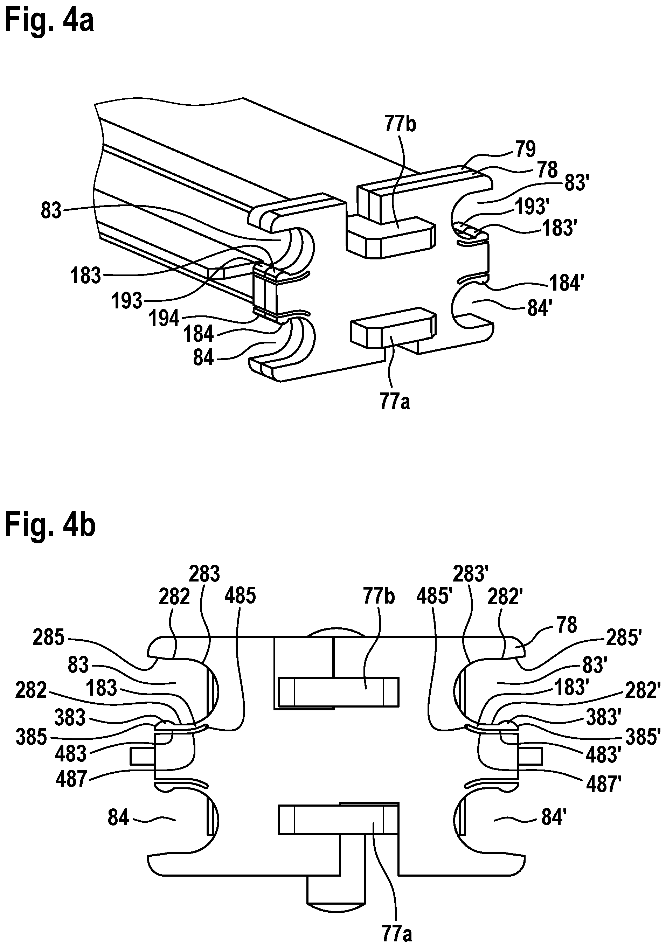

In the following, one specific cut-out, respectively the cut-out 83, shall be described in detail, whereas the cut-outs 83', 84 and 84' are built similarly (compare also Fig. 4a) :
Frame plate 78 and frame plate 79 feature a lever 183 and 193 located at the respective rim of the cut-out 83 and thus forming a flexible element towards the size of the cut-out 83. The lever 183 is formed by a cut 483 and a cut 485, connected by an edge 487 and thus forming an angle to form the lever 183 along the rim of the cut-out 83. Toward the open side of the cut-out 83, the lever 183 features a straight 382, a rise 383 and a chamfer 385, while a circular section 283 of the cut-out 83 on an opposite side of the lever 183 followed by a straight 282' ends in a chamfer 285. The chamfers 285 and 385 thus enable for entering the block 51 into the cut-out 83 and therefore deforming the lever 183 by flexibly pushing the rise 383 out of the way of the block 51. As the block 51 is located completely in the cut-out 83, the rise 383, which is protruding into the cut-out 83, holds the block 51 in place. The same function applies to the cut-outs 83', 84 and 84' as to be seen in Fig. 4b.

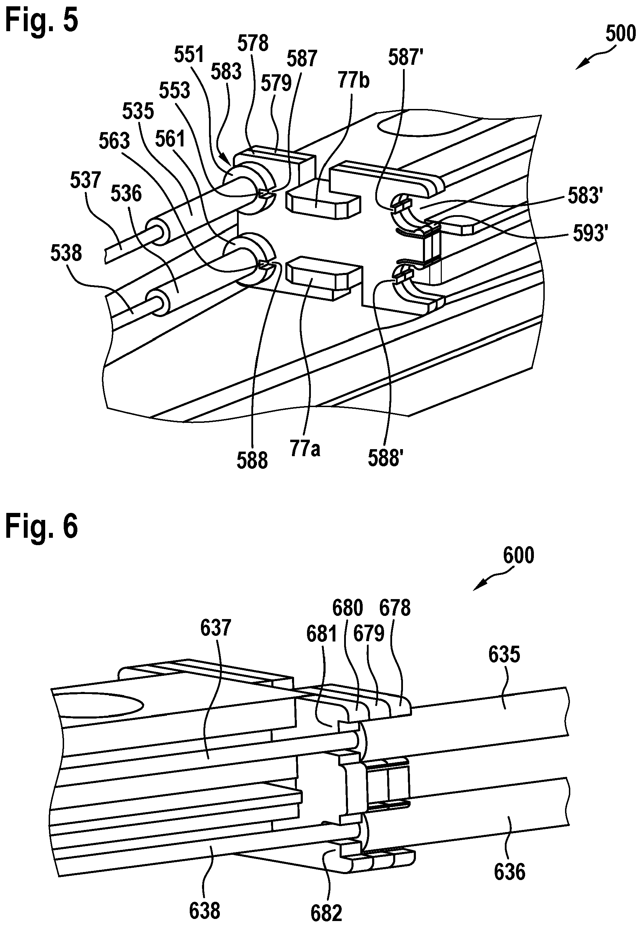

Another holder 500, which is suitable to replace the holder 100 on the deflection control mechanism 29 features slightly different cut-outs 583, 583', 584, 584'. In comparison to the previous example, each cut-out, as an example described on cut-out 583', features a ridge 587' to be engaged with a respective notch on a bushing 551, which is shown in connection with the cut-out 583 in Fig. 5. This bushing 551 features the notch 553, which engages with the ridge 587 on the respective place in respective frame plates 578 and 579. Sheaths 535 and 536 as well as control wires 537 and 538 are similar to the above-mentioned sheaths 35 and 36 and the respective control wires 37 and 38.

Another holder 600, the function of which is similar to the two above-mentioned examples, features a frame plate 678, a frame plate 679 and a frame plate 680, each stacked and linked with the hooks 77a and 77b of the main structural parts of the deflection control mechanism 29. In contrast to the above-mentioned examples, only the frame plates 678 and 679 feature levers and thus flexible means to accept and holds sheaths 635 and 636 (compare Fig. 6). The frame plate 380 features a stop 681 and a stop 682 as well as two more stops on the opposite side (not shown in Fig. 6) to form a longitudinal stop for the sheaths 635 and 636 and therefore present an option without the blocks and/or bushings in the above-mentioned examples. A cover 690, which can be bent over the main structure of the deflection control mechanism 29, features a flap 692 with notches 693 and 695, wherein the notches engage with the sheaths 635 and 636 of the respective control wires 637 and 638 to deform the sheaths 635 and 636, respectively, and thus prevent a rotation motion of each sheath against the main structure of the deflection control mechanism.

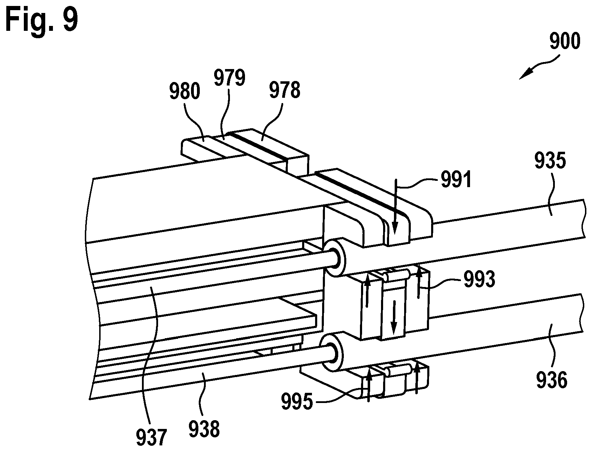

Yet another holder 900 to hold sheaths 935 and 936 (compare Fig. 9) features frame plates 978 and 979 and 980, wherein frame plate 979 features levers according to the above-mentioned examples and frame plate 978 and 980 feature U-shaped cut-outs. Once the sheath 935 or 936 is engaged in the frame plate 979 by means of the flexible lever and elastic forces, the frame plates can, for example by means of a screwing mechanism, be moved along the directions 991, 993 and 995, thus shifting the frame plates against each other and thus finally clamping down each sheath 935 and 936 in place.

### Reference numerals

- 10: Flexible endoscope
- 20: Handpiece
- 21: Housing
- 22: Hand wheel
- 23: Hand wheel
- 24: Knob
- 25: Button
- 26: Connector
- 27: Light cable
- 28: Instrument port
- 29: Deflection control mechanism
- 30: Shaft
- 31: Steerable section
- 32: End cap
- 33: Pivoting element
- 35: Sheath
- 35': Sheath
- 36: Sheath
- 36': Sheath
- 37: Control wire
- 37': Control wire
- 38: Control wire
- 38': Control wire
- 40: Body
- 41: Seal ring
- 42: Lever
- 43: Axle
- 44: Cage
- 50: Upper layer
- 51: Bushing
- 51': Bushing
- 52: Block
- 52': Block
- 56: Block
- 56': Block
- 58: Chain
- 61: Bushing
- 61': Bushing
- 62: Block
- 62': Block
- 66: Block
- 66': Block
- 68: Chain
- 70: Plastic plate
- 77a: Hook
- 77b: Hook
- 78: Frame plate
- 79: Frame plate
- 83: Cutout
- 83': Cutout
- 84: Cutout
- 84': Cutout
- 90: Cover
- 100: Holder
- 183: Lever
- 183': Lever
- 184: Lever
- 184': Lever
- 193: Lever
- 193': Lever
- 194: Lever
- 194': Lever
- 282: Straight
- 282': Straight
- 283: Circle
- 283': Circle
- 285: Chamfer
- 285': Chamfer
- 383: Rise
- 385: Chamfer
- 383': Rise
- 385': Chamfer
- 483: Cut
- 485: Cut
- 487: Edge
- 483': Cut
- 485': Cut
- 487': Edge
- 500: Holder
- 535: Sheath
- 536: Sheath
- 537: Control wire
- 538: Control wire
- 551: Bushing
- 553: Notch
- 561: Bushing
- 563: Notch
- 578: Frame plate
- 579: Frame plate
- 583: Cutout
- 583': Cutout
- 584: Cutout
- 584': Cutout
- 587: Ridge
- 587': Ridge
- 588: Ridge
- 588': Ridge
- 593': Lever
- 600: Holder
- 635: Sheath
- 636: Sheath
- 637: Control wire
- 638: Control wire
- 678: Frame plate
- 679: Frame plate
- 680: Frame plate
- 681: Stop
- 682: Stop
- 690: Cover
- 692: Flap
- 693: Notch
- 695: Notch
- 900: Holder
- 935: Sheath
- 936: Sheath
- 937: Control wire
- 938: Control wire
- 978: Frame plate
- 979: Frame plate
- 980: Frame plate
- 991: Direction
- 993: Direction
- 995: Direction

## Claims

1. Deflection control mechanism (29) for a medical device (10), particularly for a steerable flexible endoscope (10), the endoscope (10) having an elongate flexible shaft (30) comprising a steerable section (31) that is deflectable by movement of at least one pair of counteracting control wires (37, 37', 38, 38', 537, 537', 538, 538', 637, 637', 638, 638', 937, 938), the deflection control mechanism comprising a support structure (40, 44, 70), a drive wheel (22, 23) rotatably supported in or on the support structure (40, 44, 70), and an elongate traction element (58, 68) having a flexible section, a first coupling section connected to a first end of the flexible section and a second coupling section connected to a second end of the flexible section, wherein the flexible section of the traction element mechanically engages with the drive wheel (22, 23), wherein the first and second coupling sections each comprise a fixation element (56, 56', 66, 66') for fixing a respective proximal end of each control wire (37, 37', 38, 38', 537, 537', 538, 538', 637, 637', 638, 638', 937, 938) of the at least one pair of control wires (37, 37', 38, 38', 537, 537', 538, 538', 637, 637', 638, 638', 937, 938), whereas each control wire (37, 37', 38, 38', 537, 537', 538, 538', 637, 637', 638, 638', 937, 938) features a respective guide tube (35, 35', 36, 36', 535, 536, 635, 636, 935, 936) to guide each respective control wire (37, 37', 38, 38', 537, 537', 538, 538', 637, 637', 638, 638', 937, 938) along a longitudinal axis, the respective guide tube (35, 35', 36, 36', 535, 536, 635, 636, 935, 936) being held in a respective guide tube mount opening (83, 83', 84, 84', 583, 583', 584, 584') of a guide tube mount (78, 79, 578, 579, 678, 679, 680, 978, 979, 980) of the support structure (40, 44, 70), the guide tube mount opening (83, 83', 84, 84', 583, 583', 584, 584') having an entry opening to accept the respective guide tube (35, 35', 36, 36', 535, 536, 635, 636, 935, 936), **characterized in that** the respective guide tube mount opening (83, 83', 84, 84', 583, 583', 584, 584') features a clamping device with a respective clamping lever (183, 183', 184, 184', 193, 193', 194, 194', 593'), the clamping lever (183, 183', 184, 184', 193, 193', 194, 194', 593') being linked to the guide tube mount (78, 79, 578, 579, 678, 679, 680, 978, 979, 980) with a spring mount and thus being elastically movable against a restoring force of the spring mount in relation to the guide tube mount opening (83, 83', 84, 84', 583, 583', 584, 584') between a clamping position to hold the respective guide tube (35, 35', 36, 36', 535, 536, 635, 636, 935, 936) in the respective guide tube mount opening (83, 83', 84, 84', 583, 583', 584, 584') by reducing a size of the guide tube mount opening (83, 83', 84, 84', 583, 583', 584, 584') and/or applying the restoring force to the respective guide tube (35, 35', 36, 36', 535, 536, 635, 636, 935, 936) and a mounting position to release the respective guide tube (35, 35', 36, 36', 535, 536, 635, 636, 935, 936) from the guide tube mount opening (83, 83', 84, 84', 583, 583', 584, 584') by being elastically expanded against the restoring force to expand the size of the guide tube mount opening (83, 83', 84, 84', 583, 583', 584, 584').

2. Deflection control mechanism according to claim 1, **characterized in that** the clamping lever (183, 183', 184, 184', 193, 193', 194, 194', 593') being integrally formed into the guide tube mount (78, 79, 578, 579, 678, 679, 680, 978, 979, 980) by a gap (483, 483', 485, 485'), the gap (483, 483', 485, 485') separating a main body of the guide tube mount (78, 79, 578, 579, 678, 679, 680, 978, 979, 980) from the clamping lever (183, 183', 184, 184', 193, 193', 194, 194' , 593').

3. Deflection control mechanism according to claim 1 or 2, **characterized in that** a spring section of the clamping lever (183, 183', 184, 184', 193, 193', 194, 194', 593') forms the spring mount by using elastic material properties of a material of the clamping lever (183, 183', 184, 184', 193, 193', 194, 194', 593') and/or of the guide tube mount (78, 79, 578, 579, 678, 679, 680, 978, 979, 980).

4. Deflection control mechanism according to one of the previous claims, **characterized in that** the respective clamping lever (183, 183', 184, 184', 193, 193', 194, 194', 593') features a locking section (383, 383'), the locking section (383, 383') protruding from the clamping lever (183, 183', 184, 184', 193, 193', 194, 194', 593') into the guide tube mount opening (83, 83', 84, 84', 583, 583', 584, 584') to locally reduce the size of the guide tube mount opening (83, 83', 84, 84', 583, 583', 584, 584') for locking the respective guide tube (35, 35', 36, 36', 535, 536, 635, 636) in the clamping position.

5. Deflection control mechanism according to one of the previous claims, **characterized in that** the respective clamping lever (183, 183', 184, 184', 193, 193', 194, 194', 593') and/or the respective guide tube mount (78, 79, 578, 579, 678, 679, 680, 978, 979, 980) features a chamfer (285, 285', 385, 385') positioned at the entry opening to facilitate accepting the respective guide tube (35, 35', 36, 36', 535, 536, 635, 636, 935, 936) by reducing the necessary force for entering the respective guide tube (35, 35', 36, 36', 535, 536, 635, 636, 935, 936) into the guide tube mount opening (83, 83', 84, 84', 583, 583', 584, 584') against the restoring force.

6. Deflection control mechanism according to one of the previous claims, **characterized in that** the guide tube mount opening (83, 83', 84, 84', 583, 583', 584, 584') features an uneven geometry (282, 282') with varying radii around its circumference such that an uneven geometry of the respective guide tube (35, 35', 36, 36', 535, 536, 635, 636, 935, 936) matches the uneven geometry (282, 282') of the guide tube mount opening (83, 83', 84, 84', 583, 583', 584, 584') in the clamping position to prevent the guide tube (35, 35', 36, 36', 535, 536, 635, 636, 935, 936) from rotating around the longitudinal axis.

7. Deflection control mechanism according to one of the previous claims, **characterized in that** the respective guide tube (35, 35', 36, 36', 535, 536, 635, 636, 935, 936) features a longitudinal stop (51, 51', 61, 61', 551, 561, 681, 682) engaging with the guide tube mount in the clamping position to prevent the guide tube (35, 35', 36, 36', 535, 536, 635, 636, 935, 936) from moving in relation to the guide tube mount along the longitudinal axis.

8. Deflection control mechanism according to one of the previous claims, **characterized in that** the guide tube (35, 35', 36, 36', 535, 536, 635, 636, 935, 936) features a locking piece (51, 51', 61, 61', 551, 561, 681, 682) being mechanically attached to the guide tube (35, 35', 36, 36', 535, 536, 635, 636, 935, 936), especially presenting the uneven geometry of the guide tube (35, 35', 36, 36', 535, 536, 635, 636, 935, 936) and/or the longitudinal stop (51, 51', 61, 61', 551, 561, 681, 682).

9. Deflection control mechanism according to one of the previous claims, **characterized in that** the guide tube mount (78, 79, 578, 579, 678, 679, 680, 978, 979, 980) is formed into a first mount plate (78, 578, 678, 978), which is especially positioned essentially orthogonally in relation to the longitudinal axis, whereas the guide tube mount (78, 79, 578, 579, 678, 679, 680, 978, 979, 980) especially features a second mount plate (79, 579, 679, 979), a third mount plate (680, 980) and/or another mount plate, each positioned essentially orthogonally in relation to the longitudinal axis and forming the guide tube mount (78, 79, 578, 579, 678, 679, 680, 978, 979, 980), the uneven geometry (282, 282') and/or the longitudinal stop (51, 51', 61, 61', 551, 561, 681, 682).

10. Deflection control mechanism according to one of the previous claims, **characterized in that** the support structure (40, 44, 70) features support structure elements (40, 44, 70) with hooking means (77a, 77b) to accept the guide tube mount (78, 79, 578, 579, 678, 679, 680, 978, 979, 980), especially the first mount plate (78, 578, 678, 978), the second mount plate (79, 579, 679, 979), the third mount plate (680, 980) and/or the other mount plate, whereas the hooking means (77a, 77b) especially accept the guide tube mount (78, 79, 578, 579, 678, 679, 680, 978, 979, 980), especially the first mount plate (78, 578, 678, 978), the second mount plate (79, 579, 679, 979), the third mount plate (680, 980) and/or the other mount plate in a hooking axis being essentially positioned in parallel to a mount plane of the guide tube mount (78, 79, 578, 579, 678, 679, 680, 978, 979, 980), the first mount plate (78, 578, 678, 978), the second mount plate (79, 579, 679, 979), the third mount plate (680, 980) and/or the other mount plate.

11. Deflection control mechanism according to one of the previous claims, **characterized in that** the guide tube mount (78, 79, 578, 579, 678, 679, 680, 978, 979, 980), the first mount plate (78, 578, 678, 978), the second mount plate (79, 579, 679, 979), the third mount plate (680, 980) and/or the other mount plate and/or the support structure elements (40, 44, 70) is and/or are manufactured from steel, especially from stainless steel and/or from sheet material, especially cut via laser cutting and/or waterjet cutting.

12. Deflection control mechanism according to one of the previous claims, **characterized in that** the support structure (40, 44, 70) features a cover (90), the cover (90) engaging with the support structure (40, 44, 70) in a covering position to protect the support structure (40, 44, 70), the elongate traction element (58, 58, 68, 68'), the first coupling section, the second coupling section, the respective fixation element (56, 56', 66, 66') and/or a respective control wire (37, 37', 38, 38', 537, 537', 538, 538', 637, 637', 638, 638', 937, 938) and/or fixing the support elements and/or the guide tube mount (78, 79, 578, 579, 678, 679, 680, 978, 979, 980), the first mount plate (78, 578, 678, 978), the second mount plate (79, 579, 679, 979), the third mount plate (680, 980) and/or the other mount plate in place, whereas especially the respective guide tube (35, 35', 36, 36', 535, 536, 635, 636, 935, 936) or the respective guide tubes (35, 35', 36, 36', 535, 536, 635, 636, 935, 936) and/or the locking piece (51, 51', 61, 61', 551, 561, 681, 682) is held in its position in the clamping position by the cover (90) in the covering position.

13. Medical device, particularly steerable flexible endoscope with a deflection control mechanism according to one of the claims above.

14. Method for servicing a deflection control mechanism or a medical device according to one of the claims above, **comprising** the following steps:
- Disengaging the respective guide tube (35, 35', 36, 36', 535, 536, 635, 636, 935, 936) from the respective guide tube mount opening (83, 83', 84, 84', 583, 583', 584, 584') from the clamping position by retracting the respective guide tube (35, 35', 36, 36', 535, 536, 635, 636, 935, 936) against the restoring force such that the respective guide tube (35, 35', 36, 36', 535, 536, 635, 636, 935, 936) is free from the respective guide tube mount,
- Manipulating the respective fixation element and/or the respective control wire (37, 37', 38, 38', 537, 537', 538, 538', 637, 637', 638, 638', 937, 938) according to a service operation, such that the service operation has been performed,
- Reengaging the respective guide tube with the respective guide tube mount opening (83, 83', 84, 84', 583, 583', 584, 584') into the clamping position by pushing the respective guide tube (35, 35', 36, 36', 535, 536, 635, 636) into the respective guide tube mount opening (83, 83', 84, 84', 583, 583', 584, 584') against the restoring force such that the respective guide tube (35, 35', 36, 36', 535, 536, 635, 636, 935, 936) is brought into the clamping position and thus being fixed in the respective guide tube mount opening (83, 83', 84, 84', 583, 583', 584, 584'),
such that the deflection control mechanism or the medical device is being serviced.

15. Method for assembling a deflection control mechanism according to one of the claims 1 to 12 or a medical device according to claim 13, **comprising** the following steps:
- Presenting the support structure (40, 44, 70), at least one guide tube (35, 35', 36, 36', 535, 536, 635, 636, 935, 936) and the guide tube mount, such that the support structure (40, 44, 70), at least one guide tube (35, 35', 36, 36', 535, 536, 635, 636, 935, 936) and the guide tube mount are present,
- Engaging the respective guide tube (35, 35', 36, 36', 535, 536, 635, 636, 935, 936) with the respective guide tube mount opening (83, 83', 84, 84', 583, 583', 584, 584') into the clamping position by pushing the respective guide tube (35, 35', 36, 36', 535, 536, 635, 636, 935, 936) into the respective guide tube mount opening (83, 83', 84, 84', 583, 583', 584, 584') against the restoring force such that the respective guide tube (35, 35', 36, 36', 535, 536, 635, 636, 935, 936) is brought into the clamping position and thus being fixed in the respective guide tube mount opening (83, 83', 84, 84', 583, 583', 584, 584'),
such that the deflection control mechanism or the medical device is assembled.

## Patentansprüche

1. Ablenksteuerungsmechanismus (29) für ein medizinisches Gerät (10), insbesondere für ein lenkbares flexibles Endoskop (10), wobei das Endoskop (10) einen länglichen flexiblen Schaft (30) aufweist, der einen lenkbaren Abschnitt (31) umfasst, der durch die Bewegung von mindestens einem Paar gegenwirkender Steuerdrähte (37, 37', 38, 38', 537, 537', 538, 538', 637, 637', 638, 638', 937, 938) abgelenkt werden kann, wobei der Ablenksteuerungsmechanismus eine Stützstruktur (40, 44, 70), ein Antriebsrad (22, 23), das drehbar in oder an der Stützstruktur (40, 44, 70) gestützt ist, und ein längliches Zugelement (58, 68) mit einem flexiblen Abschnitt, einem ersten Koppelabschnitt, der mit einem ersten Ende des flexiblen Abschnitts verbunden ist, und einem zweiten Koppelabschnitt, der mit einem zweiten Ende des flexiblen Abschnitts verbunden ist, umfasst, wobei der flexible Abschnitt des Zugelements mechanisch mit dem Antriebsrad (22, 23) in Eingriff steht, wobei der erste und der zweite Koppelabschnitt jeweils ein Fixierelement (56, 56', 66, 66') zum Fixieren eines jeweiligen proximalen Endes jedes Steuerdrahts (37, 37', 38, 38', 537, 537', 538, 538', 637, 637', 638, 638', 937, 938) des mindestens einen Paars von Steuerdrähten (37, 37', 38, 38', 537, 537', 538, 538', 637, 637', 638, 638', 937, 938) umfassen, wobei jeder Steuerdraht (37, 37', 38, 38', 537, 537', 538, 538', 637, 637', 638, 638', 937, 938) über ein jeweiliges Führungsrohr (35, 35', 36, 36', 535, 536, 635, 636, 935, 936) verfügt, um jeden jeweiligen Steuerdraht (37, 37', 38, 38', 537, 537', 538, 538', 637, 637', 638, 638', 937, 938) entlang einer Längsachse zu führen, wobei das jeweilige Führungsrohr (35, 35', 36, 36', 535, 536, 635, 636, 935, 936) in einer jeweiligen Führungsrohrhalterungsöffnung (83, 83', 84, 84', 583, 583', 584, 584') einer Führungsrohrhalterung (78, 79, 578, 579, 678, 679, 680, 978, 979, 980) der Stützstruktur (40, 44, 70) gehalten wird, wobei die Führungsrohrhalterungsöffnung (83, 83', 84, 84', 583, 583', 584, 584') eine Eintrittsöffnung zur Aufnahme des jeweiligen Führungsrohrs (35, 35', 36, 36', 535, 536, 635, 636, 935, 936) aufweist, **dadurch gekennzeichnet, dass** die jeweilige Führungsrohrhalterungsöffnung (83, 83', 84, 84', 583, 583', 584, 584') über ein Klemmgerät mit einem jeweiligen Klemmhebel (183, 183', 184, 184', 193, 193', 194, 194', 593') verfügt, wobei der Klemmhebel (183, 183', 184, 184', 193, 193', 194, 194', 593') mit der Führungsrohrhalterung (78, 79, 578, 579, 678, 679, 680, 978, 979, 980) über eine Federhalterung verknüpft ist und somit gegen eine Rückstellkraft der Federhalterung in Bezug auf die Führungsrohrhalterungsöffnung (83, 83', 84, 84', 583, 583', 584, 584') zwischen einer Klemmposition zum Halten des jeweiligen Führungsrohrs (35, 35', 36, 36', 535, 536, 635, 636, 935, 936) in der jeweiligen Führungsrohrhalterungsöffnung (83, 83', 84, 84', 583, 583', 584, 584') indem eine Größe der Führungsrohrhalterungsöffnung (83, 83', 84, 84', 583, 583', 584, 584') reduziert wird und/oder die Rückstellkraft auf das jeweilige Führungsrohr (35, 35', 36, 36', 535, 536, 635, 636, 935, 936) ausgeübt wird und einer Montageposition elastisch beweglich ist, um das jeweilige Führungsrohr (35, 35', 36, 36', 535, 536, 635, 636, 935, 936) aus der Führungsrohrhalterungsöffnung (83, 83', 84, 84', 583, 583', 584, 584') durch elastische Dehnung gegen die Rückstellkraft freizugeben, um die Größe der Führungsrohrhalterungsöffnung (83, 83', 84, 84', 583, 583', 584, 584') zu dehnen.

2. Ablenksteuerungsmechanismus nach Anspruch 1, **dadurch gekennzeichnet, dass** der Klemmhebel (183, 183', 184, 184', 193, 193', 194, 194', 593') durch einen Spalt (483, 483', 485, 485') einstückig in die Führungsrohrhalterung (78, 79, 578, 579, 678, 679, 680, 978, 979, 980) ausgebildet ist, wobei der Spalt (483, 483', 485, 485') einen Hauptkörper der Führungsrohrhalterung (78, 79, 578, 579, 678, 679, 680, 978, 979, 980) vom Klemmhebel (183, 183', 184, 184', 193, 193', 194, 194', 593') trennt.

3. Ablenksteuerungsmechanismus nach Anspruch 1 oder 2, **dadurch gekennzeichnet, dass** ein Federabschnitt des Klemmhebels (183, 183', 184, 184', 193, 193', 194, 194', 593') die Federhalterung bildet, indem er die elastischen Materialeigenschaften eines Materials des Klemmhebels (183, 183', 184, 184', 193, 193', 194, 194', 593') und/oder der Führungsrohrhalterung (78, 79, 578, 579, 678, 679, 680, 978, 979, 980) verwendet.

4. Ablenksteuerungsmechanismus nach einem der vorhergehenden Ansprüche, **dadurch gekennzeichnet, dass** der jeweilige Klemmhebel (183, 183', 184, 184', 193, 193', 194, 194', 593') über einen Verriegelungsabschnitt (383, 383') verfügt, wobei der Verriegelungsabschnitt (383, 383') vom Klemmhebel (183, 183', 184, 184', 193, 193', 194, 194', 593') in die Führungsrohrhalterungsöffnung (83, 83', 84, 84', 583, 583', 584, 584') ragt, um die Größe der Führungsrohrhalterungsöffnung (83, 83', 84, 84', 583, 583', 584, 584') lokal zu reduzieren, um das jeweilige Führungsrohr (35, 35', 36, 36', 535, 536, 635, 636) in der Klemmposition zu verriegeln.

5. Ablenksteuerungsmechanismus nach einem der vorhergehenden Ansprüche, **dadurch gekennzeichnet, dass** der jeweilige Klemmhebel (183, 183', 184, 184', 193, 193', 194, 194', 593') und/oder die jeweilige Führungsrohrhalterung (78, 79, 578, 579, 678, 679, 680, 978, 979, 980) über eine Fase (285, 285', 385, 385') verfügt, die an der Eintrittsöffnung positioniert ist, um die Aufnahme des jeweiligen Führungsrohrs (35, 35', 36, 36', 535, 536, 635, 636, 935, 936) zu erleichtern, indem die erforderliche Kraft zum Einführen des jeweiligen Führungsrohrs (35, 35', 36, 36', 535, 536, 635, 636, 935, 936) in die Führungsrohrhalterungsöffnung (83, 83', 84, 84', 583, 583', 584, 584') gegen die Rückstellkraft reduziert wird.

6. Ablenksteuerungsmechanismus nach einem der vorhergehenden Ansprüche, **dadurch gekennzeichnet, dass** die Führungsrohrhalterungsöffnung (83, 83', 84, 84', 583, 583', 584, 584') über eine ungleichmäßige Geometrie (282, 282') mit variierenden Radien um ihren Umfang verfügt, sodass eine ungleichmäßige Geometrie des jeweiligen Führungsrohrs (35, 35', 36, 36', 535, 536, 635, 636, 935, 936) mit der ungleichmäßigen Geometrie (282, 282') der Führungsrohrhalterungsöffnung (83, 83', 84, 84', 583, 583', 584, 584') in der Klemmposition übereinstimmt, um zu verhindern, dass sich das Führungsrohr (35, 35', 36, 36', 535, 536, 635, 636, 935, 936) um die Längsachse dreht.

7. Ablenksteuerungsmechanismus nach einem der vorhergehenden Ansprüche, **dadurch gekennzeichnet, dass** das jeweilige Führungsrohr (35, 35', 36, 36', 535, 536, 635, 636, 935, 936) über einen Längsanschlag (51, 51', 61, 61', 551, 561, 681, 682) verfügt, der in der Klemmposition mit der Führungsrohrhalterung in Eingriff steht, um zu verhindern, dass sich das Führungsrohr (35, 35', 36, 36', 535, 536, 635, 636, 935, 936) in Bezug auf die Führungsrohrhalterung entlang der Längsachse bewegt.

8. Ablenksteuerungsmechanismus nach einem der vorhergehenden Ansprüche, **dadurch gekennzeichnet, dass** das Führungsrohr (35, 35', 36, 36', 535, 536, 635, 636, 935, 936) über ein Verriegelungsstück (51, 51', 61, 61', 551, 561, 681, 682) verfügt, das mechanisch am Führungsrohr (35, 35', 36, 36', 535, 536, 635, 636, 935, 936) angebracht ist, insbesondere die ungleichmäßige Geometrie des Führungsrohrs (35, 35', 36, 36', 535, 536, 635, 636, 935, 936) und/oder den Längsanschlag (51, 51', 61, 61', 551, 561, 681, 682) präsentierend.

9. Ablenksteuerungsmechanismus nach einem der vorhergehenden Ansprüche, **dadurch gekennzeichnet, dass** die Führungsrohrhalterung (78, 79, 578, 579, 678, 679, 680, 978, 979, 980) zu einer ersten Halterungsplatte (78, 578, 678, 978) ausgebildet ist, die insbesondere im Wesentlichen orthogonal in Bezug auf die Längsachse angeordnet ist, wobei die Führungsrohrhalterung (78, 79, 578, 579, 678, 679, 680, 978, 979, 980) insbesondere über eine zweite Halterungsplatte (79, 579, 679, 979), eine dritte Halterungsplatte (680, 980) und/oder eine weitere Halterungsplatte verfügt, die jeweils im Wesentlichen orthogonal in Bezug auf die Längsachse angeordnet sind und die Führungsrohrhalterung (78, 79, 578, 579, 678, 679, 680, 978, 979, 980), die ungleichmäßige Geometrie (282, 282') und/oder den Längsanschlag (51, 51', 61, 61', 551, 561, 681, 682) bilden.

10. Ablenksteuerungsmechanismus nach einem der vorhergehenden Ansprüche, **dadurch gekennzeichnet, dass** die Stützstruktur (40, 44, 70) über Stützstrukturelemente (40, 44, 70) mit Einhakmitteln (77a, 77b) zur Aufnahme der Führungsrohrhalterung (78, 79, 578, 579, 678, 679, 680, 978, 979, 980), insbesondere der ersten Halterungsplatte (78, 578, 678, 978), der zweiten Halterungsplatte (79, 579, 679, 979), der dritten Halterungsplatte (680, 980) und/oder der weiteren Halterungsplatte verfügt, wobei die Einhakmittel (77a, 77b) insbesondere die Führungsrohrhalterung (78, 79, 578, 579, 678, 679, 680, 978, 979, 980), insbesondere die erste Halterungsplatte (78, 578, 678, 978), die zweite Halterungsplatte (79, 579, 679, 979), die dritte Halterungsplatte (680, 980) und/oder die weitere Halterungsplatte in einer Einhakachse aufnehmen, die im Wesentlichen parallel zu einer Halterungsebene der Führungsrohrhalterung (78, 79, 578, 579, 678, 679, 680, 978, 979, 980), der ersten Halterungsplatte (78, 578, 678, 978), der zweiten Halterungsplatte (79, 579, 679, 979), der dritten Halterungsplatte (680, 980) und/oder der weiteren Halterungsplatte positioniert ist.

11. Ablenksteuerungsmechanismus nach einem der vorhergehenden Ansprüche, **dadurch gekennzeichnet, dass** die Führungsrohrhalterung (78, 79, 578, 579, 678, 679, 680, 978, 979, 980), die erste Halterungsplatte (78, 578, 678, 978), die zweite Halterungsplatte (79, 579, 679, 979), die dritte Halterungsplatte (680, 980) und/oder die weitere Halterungsplatte und/oder die Stützstrukturelemente (40, 44, 70) aus Stahl, insbesondere aus Edelstahl und/oder aus Blechmaterial, hergestellt ist und/oder sind, insbesondere durch Laserschneiden und/oder Wasserstrahlschneiden geschnitten.

12. Ablenksteuerungsmechanismus nach einem der vorhergehenden Ansprüche, **dadurch gekennzeichnet, dass** die Stützstruktur (40, 44, 70) über eine Abdeckung (90) verfügt, wobei die Abdeckung (90) in einer Abdeckposition mit der Stützstruktur (40, 44, 70) in Eingriff steht, um die Stützstruktur (40, 44, 70), das längliche Zugelement (58, 58, 68, 68'), den ersten Koppelabschnitt, den zweiten Koppelabschnitt, das jeweilige Fixierelement (56, 56', 66, 66') und/oder einen jeweiligen Steuerdraht (37, 37', 38, 38', 537, 537', 538, 538', 637, 637', 638, 638', 937, 938) zu schützen und/oder die Stützelemente und/oder die Führungsrohrhalterung (78, 79, 578, 579, 678, 679, 680, 978, 979, 980), die erste Halterungsplatte (78, 578, 678, 978), die zweite Halterungsplatte (79, 579, 679, 979), die dritte Halterungsplatte (680, 980) und/oder die weitere Halterungsplatte an Ort und Stelle zu fixieren, wobei insbesondere das jeweilige Führungsrohr (35, 35', 36, 36', 535, 536, 635, 636, 935, 936) oder die jeweiligen Führungsrohre (35, 35', 36, 36', 535, 536, 635, 636, 935, 936) und/oder das Verriegelungsstück (51, 51', 61, 61', 551, 561, 681, 682) von der Abdeckung (90) in der Abdeckposition in ihrer Position in der Klemmposition gehalten wird.

13. Medizinisches Gerät, insbesondere lenkbares flexibles Endoskop mit einem Ablenksteuerungsmechanismus nach einem der vorstehenden Ansprüche.

14. Verfahren zur Wartung eines Ablenksteuerungsmechanismus oder eines medizinischen Geräts nach einem der vorstehenden Ansprüche, **umfassend** die folgenden Schritte:
- Lösen des jeweiligen Führungsrohrs (35, 35', 36, 36', 535, 536, 635, 636, 935, 936) aus der jeweiligen Führungsrohrhalterungsöffnung (83, 83', 84, 84', 583, 583', 584, 584') aus der Klemmposition durch Zurückziehen des jeweiligen Führungsrohrs (35, 35', 36, 36', 535, 536, 635, 636, 935, 936) gegen die Rückstellkraft, sodass das jeweilige Führungsrohr (35, 35', 36, 36', 535, 536, 635, 636, 935, 936) frei von der jeweiligen Führungsrohrhalterung ist,
- Manipulieren des jeweiligen Fixierelements und/oder des jeweiligen Steuerdrahts (37, 37', 38, 38', 537, 537', 538, 538', 637, 637', 638, 638', 937, 938) gemäß einem Wartungsvorgang, sodass der Wartungsvorgang durchgeführt wurde,
- Erneutes Eingreifen des jeweiligen Führungsrohrs in die jeweilige Führungsrohrhalterungsöffnung (83, 83', 84, 84', 583, 583', 584, 584') in die Klemmposition, indem das jeweilige Führungsrohr (35, 35', 36, 36', 535, 536, 635, 636) gegen die Rückstellkraft in die jeweilige Führungsrohrhalterungsöffnung (83, 83', 84, 84', 583, 583', 584, 584') geschoben wird, sodass das jeweilige Führungsrohr (35, 35', 36, 36', 535, 536, 635, 636, 935, 936) in die Klemmposition gebracht und somit in der jeweiligen Führungsrohrhalterungsöffnung (83, 83', 84, 84', 583, 583', 584, 584') fixiert wird,
derart, dass der Ablenksteuerungsmechanismus oder das medizinische Gerät gewartet wird.

15. Verfahren zum Zusammenbau eines Ablenksteuerungsmechanismus nach einem der Ansprüche 1 bis 12 oder eines medizinischen Geräts nach Anspruch 13, **umfassend** die folgenden Schritte:
- Vorlegen der Stützstruktur (40, 44, 70), mindestens eines Führungsrohrs (35, 35', 36, 36', 535, 536, 635, 636, 935, 936) und der Führungsrohrhalterung, sodass die Stützstruktur (40, 44, 70), mindestens ein Führungsrohr (35, 35', 36, 36', 535, 536, 635, 636, 935, 936) und die Führungsrohrhalterung vorhanden sind,
- Eingreifen des jeweiligen Führungsrohrs (35, 35', 36, 36', 535, 536, 635, 636, 935, 936) in die jeweilige Führungsrohrhalterungsöffnung (83, 83', 84, 84', 583, 583', 584, 584') in die Klemmposition, indem das jeweilige Führungsrohr (35, 35', 36, 36', 535, 536, 635, 636, 935, 936) gegen die Rückstellkraft in die jeweilige Führungsrohrhalterungsöffnung (83, 83', 84, 84', 583, 583', 584, 584') geschoben wird, sodass das jeweilige Führungsrohr (35, 35', 36, 36', 535, 536, 635, 636, 935, 936) in die Klemmposition gebracht und somit in der jeweiligen Führungsrohrhalterungsöffnung (83, 83', 84, 84', 583, 583', 584, 584') fixiert wird,
sodass der Ablenksteuerungsmechanismus oder das medizinische Gerät zusammengebaut ist.

## Revendications

1. Mécanisme de commande de déviation (29) pour un dispositif médical (10), en particulier pour un endoscope (10) flexible orientable, l'endoscope (10) ayant une tige flexible allongée (30) comprenant une section orientable (31) qui peut être déviée par le déplacement d'au moins une paire de fils de commande antagonistes (37, 37', 38, 38', 537, 537', 538, 538', 637, 637', 638, 638', 937, 938), le mécanisme de commande de déviation comprenant une structure de support (40, 44, 70), une roue d'entraînement (22, 23) supportée de manière rotative dans ou sur la structure de support (40, 44, 70), et un élément de traction allongé (58, 68) ayant une section flexible, une première section d'accouplement reliée à une première extrémité de la section flexible et une seconde section d'accouplement reliée à une seconde extrémité de la section flexible, dans lequel la section flexible de l'élément de traction vient mécaniquement en prise avec la roue d'entraînement (22, 23), dans lequel les première et seconde sections d'accouplement comprennent chacune un élément de fixation (56, 56', 66, 66') pour fixer une extrémité proximale respective de chaque fil de commande (37, 37', 38, 38', 537, 537', 538, 538', 637, 637', 638, 638', 937, 938) de l'au moins une paire de fils de commande (37, 37', 38, 38', 537, 537', 538, 538', 637, 637', 638, 638', 937, 938), tandis que chaque fil de commande (37, 37', 38, 38', 537, 537', 538, 538', 637, 637', 638, 638', 937, 938) comporte un tube de guidage (35, 35', 36, 36', 535, 536, 635, 636, 935, 936) respectif pour guider chaque fil de commande (37, 37', 38, 38', 537, 537', 538, 538', 637, 637', 638, 638', 937, 938) respectif le long d'un axe longitudinal, le tube de guidage (35, 35', 36, 36', 535, 536, 635, 636, 935, 936) respectif étant maintenu dans une ouverture de monture de tube de guidage (83, 83', 84, 84', 583, 583', 584, 584') respective d'une monture de tube de guidage (78, 79, 578, 579, 678, 679, 680, 978, 979, 980) de la structure de support (40, 44, 70), l'ouverture de monture de tube de guidage (83, 83', 84, 84', 583, 583', 584, 584') ayant une ouverture d'entrée pour recevoir le tube de guidage (35, 35', 36, 36', 535, 536, 635, 636, 935, 936) respectif, **caractérisé en ce que** l'ouverture de monture de tube de guidage (83, 83', 84, 84', 583, 583', 584, 584') respective comporte un dispositif de serrage avec un levier de serrage (183, 183', 184, 184', 193, 193', 194, 194', 593') respectif, le levier de serrage (183, 183', 184, 184', 193, 193', 194, 194', 593') étant relié à la monture de tube de guidage (78, 79, 578, 579, 678, 679, 680, 978, 979, 980) par une monture à ressort et étant ainsi mobile élastiquement contre une force de rappel de la monture à ressort par rapport à l'ouverture de monture de tube de guidage (83, 83', 84, 84', 583, 583', 584, 584') entre une position de serrage pour maintenir le tube de guidage (35, 35', 36, 36', 535, 536, 635, 636, 935, 936) respectif dans l'ouverture de monture de tube de guidage (83, 83', 84, 84', 583, 583', 584, 584') respective en réduisant une taille de l'ouverture de monture de tube de guidage (83, 83', 84, 84', 583, 583', 584, 584') et/ou en appliquant la force de rappel au tube de guidage (35, 35', 36, 36', 535, 536, 635, 636, 935, 936) respectif et une position de monture pour libérer le tube de guidage (35, 35', 36, 36', 535, 536, 635, 636, 935, 936) respectif de l'ouverture de monture de tube de guidage (83, 83', 84, 84', 583, 583', 584, 584') en étant augmenté élastiquement contre la force de rappel pour augmenter la taille de l'ouverture de monture de tube de guidage (83, 83', 84, 84', 583, 583', 584, 584').

2. Mécanisme de commande de déviation selon la revendication 1, **caractérisé en ce que** le levier de serrage (183, 183', 184, 184', 193, 193', 194, 194', 593') est formé d'une seule pièce dans la monture de tube de guidage (78, 79, 578, 579, 678, 679, 680, 978, 979, 980) par un espace (483, 483', 485, 485'), l'espace (483, 483', 485, 485') séparant un corps principal de la monture de tube de guidage (78, 79, 578, 579, 678, 679, 680, 978, 979, 980) du levier de serrage (183, 183', 184, 184', 193, 193', 194, 194', 593').

3. Mécanisme de commande de déviation selon la revendication 1 ou 2, **caractérisé en ce qu'**une section de ressort du levier de serrage (183, 183', 184, 184', 193, 193', 194, 194', 593') forme la monture à ressort à l'aide des propriétés matérielles élastiques d'un matériau du levier de serrage (183, 183', 184, 184', 193, 193', 194, 194', 593') et/ou de la monture de tube de guidage (78, 79, 578, 579, 678, 679, 680, 978, 979, 980).

4. Mécanisme de commande de déviation selon l'une des revendications précédentes, **caractérisé en ce que** le levier de serrage (183, 183', 184, 184', 193, 193', 194, 194', 593') respectif comporte une section de verrouillage (383, 383'), la section de verrouillage (383, 383') faisant saillie du levier de serrage (183, 183', 184, 184', 193, 193', 194, 194', 593') dans l'ouverture de monture de tube de guidage (83, 83', 84, 84', 583, 583', 584, 584') pour réduire localement la taille de l'ouverture de monture de tube de guidage (83, 83', 84, 84', 583, 583', 584, 584') pour verrouiller le tube de guidage (35, 35', 36, 36', 535, 536, 635, 636) respectif dans la position de serrage.

5. Mécanisme de commande de déviation selon l'une des revendications précédentes, **caractérisé en ce que** le levier de serrage (183, 183', 184, 184', 193, 193', 194, 194', 593') respectif et/ou la monture de tube de guidage (78, 79, 578, 579, 678, 679, 680, 978, 979, 980) respective comporte un chanfrein (285, 285', 385, 385') positionné au niveau de l'ouverture d'entrée pour faciliter l'acceptation du tube de guidage (35, 35', 36, 36', 535, 536, 635, 636, 935, 936) respectif en réduisant la force nécessaire pour faire entrer le tube de guidage (35, 35', 36, 36', 535, 536, 635, 636, 935, 936) respectif dans l'ouverture de monture de tube de guidage (83, 83', 84, 84', 583, 583', 584, 584') contre la force de rappel.

6. Mécanisme de commande de déviation selon l'une des revendications précédentes, **caractérisé en ce que** l'ouverture de monture de tube de guidage (83, 83', 84, 84', 583, 583', 584, 584') comporte une géométrie irrégulière (282, 282') avec des rayons variables autour de sa circonférence, de sorte qu'une géométrie irrégulière du tube de guidage (35, 35', 36, 36', 535, 536, 635, 636, 935, 936) respectif correspond à la géométrie irrégulière (282, 282') de l'ouverture de monture de tube de guidage (83, 83', 84, 84', 583, 583', 584, 584') dans la position de serrage pour empêcher le tube de guidage (35, 35', 36, 36', 535, 536, 635, 636, 935, 936) de tourner autour de l'axe longitudinal.

7. Mécanisme de commande de déviation selon l'une des revendications précédentes, **caractérisé en ce que** le tube de guidage (35, 35', 36, 36', 535, 536, 635, 636, 935, 936) respectif comporte une butée longitudinale (51, 51', 61, 61', 551, 561, 681, 682) qui vient en prise avec la monture de tube de guidage dans la position de serrage pour empêcher le tube de guidage (35, 35', 36, 36', 535, 536, 635, 636, 935, 936) de se déplacer par rapport à la monture de tube de guidage le long de l'axe longitudinal.

8. Mécanisme de commande de déviation selon l'une des revendications précédentes, **caractérisé en ce que** le tube de guidage (35, 35', 36, 36', 535, 536, 635, 636, 935, 936) comporte une pièce de verrouillage (51, 51', 61, 61', 551, 561, 681, 682) fixée mécaniquement au tube de guidage (35, 35', 36, 36', 535, 536, 635, 636, 935, 936), présentant notamment la géométrie irrégulière du tube de guidage (35, 35', 36, 36', 535, 536, 635, 636, 935, 936) et/ou la butée longitudinale (51, 51', 61, 61', 551, 561, 681, 682).

9. Mécanisme de commande de déviation selon l'une des revendications précédentes, **caractérisé en ce que** la monture de tube de guidage (78, 79, 578, 579, 678, 679, 680, 978, 979, 980) est formée dans une première plaque de monture (78, 578, 678, 978), qui est notamment positionnée de manière essentiellement orthogonale par rapport à l'axe longitudinal, tandis que la monture de tube de guidage (78, 79, 578, 579, 678, 679, 680, 978, 979, 980) comporte notamment une deuxième plaque de monture (79, 579, 679, 979), une troisième plaque de monture (680, 980) et/ou une autre plaque de monture, chacune étant positionnée de manière essentiellement orthogonale par rapport à l'axe longitudinal et formant la monture de tube de guidage (78, 79, 578, 579, 678, 679, 680, 978, 979, 980), la géométrie irrégulière (282, 282') et/ou la butée longitudinale (51, 51', 61, 61', 551, 561, 681, 682).

10. Mécanisme de commande de déviation selon l'une des revendications précédentes, **caractérisé en ce que** la structure de support (40, 44, 70) comporte des éléments de structure de support (40, 44, 70) ayant des moyens d'accrochage (77a, 77b) pour recevoir la monture de tube de guidage (78, 79, 578, 579, 678, 679, 680, 978, 979, 980), notamment la première plaque de monture (78, 578, 678, 978), la deuxième plaque de monture (79, 579, 679, 979), la troisième plaque de monture (680, 980) et/ou l'autre plaque de monture, tandis que les moyens d'accrochage (77a, 77b) reçoivent notamment la monture de tube de guidage (78, 79, 578, 579, 678, 679, 680, 978, 979, 980), notamment la première plaque de monture (78, 578, 678, 978), la deuxième plaque de monture (79, 579, 679, 979), la troisième plaque de monture (680, 980) et/ou l'autre plaque de monture selon un axe d'accrochage qui est essentiellement positionné parallèlement à un plan de monture de la monture de tube de guidage (78, 79, 578, 579, 678, 679, 680, 978, 979, 980), de la première plaque de monture (78, 578, 678, 978), de la deuxième plaque de monture (79, 579, 679, 979), de la troisième plaque de monture (680, 980) et/ou de l'autre plaque de monture.

11. Mécanisme de commande de déviation selon l'une des revendications précédentes, **caractérisé en ce que** la monture de tube de guidage (78, 79, 578, 579, 678, 679, 680, 978, 979, 980), la première plaque de monture (78, 578, 678, 978), la deuxième plaque de monture (79, 579, 679, 979), la troisième plaque de monture (680, 980) et/ou l'autre plaque de monture et/ou les éléments de structure de support (40, 44, 70) est et/ou sont fabriqués à partir d'acier, notamment d'acier inoxydable et/ou de tôle, notamment découpés par découpe au laser et/ou par découpe par jet d'eau.

12. Mécanisme de commande de déviation selon l'une des revendications précédentes, **caractérisé en ce que** la structure de support (40, 44, 70) comporte un couvercle (90), le couvercle (90) venant en prise avec la structure de support (40, 44, 70) dans une position de recouvrement pour protéger la structure de support (40, 44, 70), l'élément de traction allongé (58, 58, 68, 68'), la première section d'accouplement, la deuxième section d'accouplement, l'élément de fixation (56, 56', 66, 66') respectif et/ou un fil de commande (37, 37', 38, 38', 537, 537', 538, 538', 637, 637', 638, 638', 937, 938) respectif et/ou maintenant les éléments de support et/ou la monture de tube de guidage (78, 79, 578, 579, 678, 679, 680, 978, 979, 980), la première plaque de monture (78, 578, 678, 978), la deuxième plaque de monture (79, 579, 679, 979), la troisième plaque de monture (680, 980) et/ou l'autre plaque de monture en place, tandis que, notamment, le tube de guidage (35, 35', 36, 36', 535, 536, 635, 636, 935, 936) respectif ou les tubes de guidage (35, 35', 36, 36', 535, 536, 635, 636, 935, 936) respectifs et/ou la pièce de verrouillage (51, 51', 61, 61', 551, 561, 681, 682) est/sont maintenu(e)(s) dans sa/leur position dans la position de serrage par le couvercle (90) dans la position de recouvrement.

13. Dispositif médical, notamment endoscope flexible orientable, doté d'un mécanisme de commande de déviation selon l'une des revendications ci-dessus.

14. Procédé d'entretien d'un mécanisme de commande de déviation ou d'un dispositif médical selon l'une des revendications ci-dessus, **comprenant** les étapes suivantes consistant à :
- dévier le tube de guidage (35, 35', 36, 36', 535, 536, 635, 636, 935, 936) respectif de l'ouverture de monture de tube de guidage (83, 83', 84, 84', 583, 583', 584, 584') respective de la position de serrage en rétractant le tube de guidage (35, 35', 36, 36', 535, 536, 635, 636, 935, 936) respectif contre la force de rappel, de sorte que le tube de guidage (35, 35', 36, 36', 535, 536, 635, 636, 935, 936) respectif est libéré de la monture de tube de guidage respectif,
- manipuler l'élément de fixation respectif et/ou le fil de commande (37, 37', 38, 38', 537, 537', 538, 538', 637, 637', 638, 638', 937, 938) respectif selon une opération d'entretien, de sorte que l'opération d'entretien ait été effectuée,
- mettre de nouveau en prise le tube de guidage respectif avec l'ouverture de monture de tube de guidage (83, 83', 84, 84', 583, 583', 584, 584') respective dans la position de serrage en poussant le tube de guidage (35, 35', 36, 36', 535, 536, 635, 636) respectif dans l'ouverture de monture de tube de guidage (83, 83', 84, 84', 583, 583', 584, 584') respective contre la force de rappel, de sorte que le tube de guidage (35, 35', 36, 36', 535, 536, 635, 636, 935, 936) respectif est amené dans la position de serrage et ainsi fixé dans l'ouverture de monture de tube de guidage (83, 83', 84, 84', 583, 583', 584, 584') respective,
de sorte que le mécanisme de commande de déviation ou le dispositif médical est en cours d'entretien.

15. Procédé d'assemblage d'un mécanisme de commande de déviation selon l'une des revendications 1 à 12 ou d'un dispositif médical selon la revendication 13, **comprenant** les étapes suivantes consistant à :
- présenter la structure de support (40, 44, 70), au moins un tube de guidage (35, 35', 36, 36', 535, 536, 635, 636, 935, 936) et la monture de tube de guidage, de sorte que la structure de support (40, 44, 70), au moins un tube de guidage (35, 35', 36, 36', 535, 536, 635, 636, 935, 936) et la monture de tube de guidage sont présents,
- mettre en prise le tube de guidage (35, 35', 36, 36', 535, 536, 635, 636, 935, 936) respectif avec l'ouverture de monture de tube de guidage (83, 83', 84, 84', 583, 583', 584, 584') respective dans la position de serrage en poussant le tube de guidage (35, 35', 36, 36', 535, 536, 635, 636, 935, 936) respectif dans l'ouverture de monture de tube de guidage (83, 83', 84, 84', 583, 583', 584, 584') respective contre la force de rappel, de sorte que le tube de guidage (35, 35', 36, 36', 535, 536, 635, 636, 935, 936) respectif est amené dans la position de serrage et ainsi fixé dans l'ouverture de monture de tube de guidage (83, 83', 84, 84', 583, 583', 584, 584') respective,
de sorte que le mécanisme de commande de déviation ou le dispositif médical est assemblé.
